Europäisches Patentamt

European Patent Office

Office européen des brevets

(11) Publication number: 0 296 557
A2

# EUROPEAN PATENT APPLICATION

(21) Application number: 88109915.4

(22) Date of filing: 22.06.88

(51) Int. Cl.⁴: C12Q 1/68 , //C12N15/00

(30) Priority: 26.06.87 US 66553

(43) Date of publication of application:
28.12.88 Bulletin 88/52

(84) Designated Contracting States:
ES GR

(71) Applicant: E.I. DU PONT DE NEMOURS AND COMPANY
Legal Department 1007 Market Street
Wilmington Delaware 19898(US)

(72) Inventor: Adler Jr.,Karl Edwin
2 Shandel Drive
Newburyport Massachusetts 01950(US)
Inventor: Miller, Jeffrey Allan
25 Gulf Road
Derry New Hampshire 03038(US)

(74) Representative: von Kreisler, Alek,
Dipl.-Chem. et al
Patentanwälte Von Kreisler-Selting-Werner
Deichmannhaus am Hauptbahnhof
D-5000 Köln 1(DE)

(54) Affinity removal of contaminating sequences from recombinant cloned NA using capture beads.

(57) Contaminating vector sequences are removed from recombinant cloned DNA or RNA using capture beads to which is covalently attached capture vector sequences which are complementary to the contaminating sequences. Alternatively, the capture vector sequences are covalently attached to one member of a specific binding pair and the capture beads are attached to the other member of the pair. The method is generally applicable to removal of contaminating DNA or RNA sequences from recombinant, cloned DNA or RNA.

EP 0 296 557 A2

Xerox Copy Centre

# AFFINITY REMOVAL OF CONTAMINATING SEQUENCES FROM RECOMBINANT CLONED NA USING CAPTURE BEADS

## Field

This invention relates to removal of undesired nucleic acid (NA), either DNA or RNA, from desired NA. It is especially applicable to removal of contaminating vector sequences from recombinant cloned NA probes. ·

## Background

Labeled DNA and RNA probe technology offers a method for rapid, accurate detection of infectious organisms in clinical samples, as well as detection or location of other target NA sequences in experimental and clinical samples. Probes containing less than about 50-100 bases are usually produced synthetically. Larger probes usually are produced by cloning recombinant DNA comprising a probe insert in a DNA cloning vector. The procedure for making a cloned, labeled single stranded DNA (ssDNA) probe involves (a) cloning recombinant DNA comprising probe DNA insert in a DNA cloning vector, (b) excising the probe DNA from the vector by treatment with endonuclease and separating the probe DNA from the vector by gel electrophoresis, (c) labeling the probe for example, by nick translation in presence of each of the four deoxyribonucleoside triphosphates (dNTP's), at least some of which are labeled (d) denaturing the probe and vector DNA. The procedure for making a cloned, labeled single stranded RNA (ssRNA) probe involves (a) cloning recombinant DNA comprising a DNA insert, corresponding to the desired ssRNA probe, in a DNA cloning vector having a transcription promoter upstream of the insert, (b) cutting the recombinant DNA with endonuclease to produce a linear DNA template containing the promoter and the insert, (c) contacting the linear DNA template with each of the four ribonucleoside triphosphates (rNTP's), at least some of which are labeled, in presence of RNA polymerase to synthesize labeled ssRNA on the template and (d) separating the DNA and unincorporated rNTP's from the ssRNA.

In the procedure for making DNA probes, contaminating, labeled vector sequences result from the fact that gel electrophoresis does not completely separate the probe DNA from the vector DNA, even when electrophoresis is repeated several times. In the procedure for making RNA probes, contaminating, labeled vector sequences result either from incomplete linearization of the recombinant DNA, such that transcription continues into the vector region, or from adventitious transcription due to presence in the vector of RNA polymerase initiation sites other than the promoter. Buffone et al., Clin. Chem., 31:2043-4 (1985).

It is essential to remove these labeled contaminating vector sequences from the labeled ssNA probes to avoid false positives due to binding of the vector sequences to homologous sequences in the sample. This is especially important where a bacterial plasmid cloning vector such as pBR322 is used to produce a probe intended for use in analyzing a clinical sample, because of the common occurrence in body fluids, feces and tissue of bacterial DNA or related cellular sequence which binds to sequences of the vector. See, for example, Buffone et al., supra, Barbacid, Science 225:670 (1984), Hino et al., Science 225:670-1 (1984), Palva, FEMS Microbiol. Letters, 28:85-91 (19085), and Diegutis et al., J. Clin. Microbiol., 23:797-799 (1986). If there is a possibility of bacterial DNA sequences being present in a sample, the removal of even trace vector contaminants may be necessary to avoid false positives.

In the case of DNA probes, a combination of gel electrophoresis and membrane-based hybridization has been used to remove contaminating vector sequences. In this method, vector ssNA is adsorbed to capture vector ssNA immobilized on a nitrocellulose or nylon membrane. A probe DNA is first partially purified by electrophoresis, then labeled and denatured. After denaturation the probe, membrane and a buffer are placed in a plastic bag (hybridization pouch) and held for an extended period under hybridization conditions. Contaminating vector sequences in the probe are captured by the membrane-adsorbed vector ssNA. Although this method gives improved removal of vector sequences compared to electrophoresis alone, it is cumbersome and time consuming. Hybridization on the membrane is slow. The entire procedure requires about 16 hours. The membrane cannot be reused because of loss of capture ssNA from the membrane. Consequently, the procedure requires large amounts of capture vector.

Another method of preparing vector-free hybridization probes is disclosed in Galau, Gene, 34:93-98 (1983). Plasmid DNA is $^{32}$P labeled and fragmented by nick translation in presence of DNaseI. Plasmid DNA fragments are denatured and renatured with an excess of vector DNA. Probe DNA remains single stranded while vector DNA becomes double stranded. The ssDNA is then separated from the vector dsDNA on hydroxylapatite. The procedure is cumbersome and time consuming and has not been demonstrated to remove trace levels of vector contamination.

DNA and RNA have been covalently attached to bead and membrane supports and used in affinity purifications to capture desired NA. Examples of literature disclosing this are Moss et al., J. Biol. Chem., 256:12655-8 (1981), Langdale et al., Gene 36:201-210 (1985), Bünemann et al., Nucleic Acids Research, 10:7163-7180 (1982), and Bünemann et al., Nucleic Acids Research 10:7181-7196 (1985). It is believed that bead-based hybridization has not been used prior to this invention to remove undesired NA from a sample containing desired and undesired NA, in particular, to remove contaminating vector sequences from recombinant probe NA.

Summary of the Invention

This invention is a method of removing undesired ssNA from a sample containing desired and undesired ssNA in an aqueous medium. The method comprises (a) contacting the sample under hybridization conditions with capture ssNA which is complementary to the undesired ssNA and which is covalently bound to solid, water insoluble beads or to one member of a specific binding pair, (b) in the case where the capture ssNA is covalently bound to one member of a specific binding pair, contacting the sample with solid, water-insoluble beads to which is covalently bound the other member of the specific binding pair, and (c) separating the beads carrying hybridized, undesired NA from the aqueous medium. In the principal application of the invention, the capture ssNA comprises ssDNA sequences of a DNA cloning vector, the desired ssNA comprises a labeled ssDNA or ssRNA probe derived from a DNA insert which has been cloned in the vector, and the undesired ssNA comprises labeled ssDNA or ssRNA contaminating sequences derived from the vector.

The invention includes solid, water insoluble capture beads to which is covalently attached DNA sequences of a DNA cloning vector, and kits comprising the beads along with buffers and printed instructions for using the contents to remove contaminating vector sequences from labeled recombinant cloned ssNA probes.

The invention also includes compounds consisting of sequences of a DNA cloning vector covalently attached to one member of a specific binding pair, and kits comprising such DNA sequences along with solid, water insoluble capture beads to which is covalently attached the other member of the specific binding pair, buffers, and printed instructions for using the contents to remove contaminating vector sequences from labeled recombinant cloned ssNA probes.

The method of this invention provides several advantages over the membrane-based hybridization method described above for removing contaminating vector sequences from NA probes. The hybridization is faster, requiring only about one hour. The procedure is less cumbersome because the capture reagents can be manufactured and stored for use at a later date, and the hybridization can be readily carried out in a microcentrifuge tube rather than a hybridization pouch. In the case where the capture DNA is covalently attached directly to the beads, the captured DNA can be removed by denaturation and the beads can be reused several times, thus requiring much less capture DNA. In the case where the capture DNA is covalently attached to one member of a specific binding pair, the hybridization can be carried out in solution offering potentially even faster hybridization and more complete removal of contaminating vector sequences.

Details of the Invention

Beads

Many different types of solid beads can be used in this invention. The beads must be water insoluble and stable to the physical and chemical conditions to which they are subjected during linking of the capture DNA or specific binding substance (e.g., avidin) and hybridization and denaturation of the capture and undesired NA. They must also be capable of being covalently linked to the capture NA or specific binding substance in a manner which is stable to the hybridization and denaturation conditions. It is desirable that the beads exhibit low nonspecific adsorption of nucleic acids in the hybridization conditions. The beads must be capable of being separated readily from the aqueous medium following hybridization, e.g., by settling, centrifugation or application of magnetic field. Beads in the size range 1-300 microns are satisfactory for separation by settling or centrifugation. Beads in the size range 10-100 microns are preferred. In general, beads which have been used heretofore in affinity purifications by hybridization of desired nucleic acids, as described in Bünemann et al., Moss et al., Langdale et al. and Bünemann et al., supra, can be used in this invention. A preferred class of beads are composed of organic polymers having terminal amine groups, such as P-100 Bio-Gel aminoethyl polyacrylamide beads of Bio-Rad Co. Other beads which can be used include Sephadex®G-25, Sephacryl®S-500, Sephacryl® S-1000, and Sepharose®CL-2B, CL-4B and CL-6B, all products of Pharmacia Fine Chemicals, Upsala, Sweden. As described by the manufacturer, Sephadex® is a bead-formed dextran gel prepared by crosslinking selected dextran fractions with epichlorohydrin. Sephacryl® beads are formed by crosslinking allyl dextran with N,N'-methylene bisacrylamide. Sepharose® CL beads are agarose crosslinked with 2,3-dibromopropanol. Also useful is Cellex®410, a dry cellulose powder available from Bio-Rad Laboratories.

Magnetic beads can also be used, such as the particles described in Hersh, U.S. 3,933,997, Ithakissios U.S. 4,115,534, Forrest et al. U.S. 4,141,687, Mansfield et al., U.S. 4,197,337 and Chagnon, Danish application DK 2374/84. The latter are commercially available under the trade name Biomag® from Advanced Magnetics. Preferred magnetic beads are the coated CrO$_2$ particles described in Lau U.S. Application S.N. 06/841,107, allowed October 3, 1986, issue fee paid December 30, 1986. Magnetic particles having an outer layer of a silane compound with functional groups, such as 3-aminopropyl-triethoxysilane, can be utilized for covalent attachment of DNA or specific binding substance, either directly or through liner compounds, as taught in the above-cited references.

Specific Binding Pairs

Preferred specific binding pairs are biotin and avidin or streptavidin. Other pairs which can be used include antibodies and their antigens or haptens; intrinsic factor and vitamin B12; folate binding protein and folic acid; thyroxine binding globulin and thyroxine; and many others.

Covalent Binding

Various conventional chemistries can be utilized for covalent attachment of the capture NA or specific binding substance to the beads. The chemistry of choice depends upon the functional groups available on the surface of the beads and/or on the specific binding substance. Covalent attachment of capture NA to beads composed of organic polymer containing terminal amine groups or coated with compounds having free amine groups, such as 3-aminopropyl-triethoxysilane, is accomplished by the condensation of the 5'-phosphate of the NA with the amine of the bead using the water soluble carbodiimide, 1-ethyl-3,3-dimethylaminopropylcarbodiimide (EDAC) in 2-morpholinoethanesulfonic acid (MES). The basic method is described in the paper "Derivatization of Unprotected polynucleotides", Chu et al., Nucleic Acid Research 11:6513-6529 (1983). Specific binding substances containing amine groups can be attached to such beads having free surface amine groups by use of a difunctional linker such as glutaraldehyde, as described in S. Avrameas, Immunochemistry 6, 43 (1969).

Capture NA can be coupled to beads such as Sephadex®G-25, Cellex®410, and Sephacryl®S-500 via diazotization, or to beads such as Sephadex®G-25, Sepharose®CL-2B and CL-6B, and Sephacryl®S-500 and S-1000 via cyanogen bromide activation, as taught in the Bünemann et al. and Bünemann references, supra. Nucleic acids can be coupled to Cellex®410 via epoxy activation as taught in the Moss et al. reference, supra.

Capture NA can be covalently bonded to amine groups of specific binding substances by the same chemistries used to bond NA to amine groups of beads, e.g., condensation of NA 5'-phosphate groups with amine groups using EDAC in MES, as described above. Capture DNA can be covalently attached to biotin by nick translation, end-filling or end-labeling using biotinylated deoxyuridyl residues as described in Chan et al., Nucleic Acid Research, 13:8083-8901 (1985) and references cited therein. Capture DNA can be attached to protein by the method described in Renz et al., Nuclei Acids Research 12:3435-3444 (1985) and used with beads covalently attached to antibodies to the protein.

Capture NA

This invention is applicable to removal of undesired, contaminating NA from any sample. Accordingly, any NA which will hybridize with contaminating NA can be used as capture NA. A large number of DNA cloning vectors can be used for making labeled DNA and RNA probes and all of these vectors can be used to provide capture DNA for use in this invention. The invention is particularly applicable to capture DNA comprising sequences of DNA vectors of bacterial origin, such as PBR322 and vectors containing all or part of pBR322, such as pBR325 and pSP64. The latter is described in Melton et al., Nucleic Acids Research 12:7035-7056 (1984).

It is possible to use complete linearized vectors as capture DNA, but it is preferable to cut the vectors into fragments of about 100 to 1000 base pairs using one or more restriction endonucleases and attach the fragments to beads or specific binding substances. Larger NA sequences can be used, but are not preferred because they are more subject to degradation during use. Capture DNA can be denatured to provide ssDNA either before or after attachment to the beads or specific binding substances. Thus the capture DNA in the products of this invention can be either dsDNA or ssDNA.

Preparation of Probes

DNA and RNA probe starting materials for this invention can be prepared by the conventional procedures outlined in the Background portion of this specification. Synthesis and cloning of recombinant DNA are described in Maniatis et al., Molecular Cloning, A Laboratory Manual, Cold Spring Harbor Laboratory (1982). Synthesis of labeled RNA probes from recombinant cloned DNA is described in Melton et al., supra. Labeling of recombinant cloned DNA with $^{32}$P by nick translation is described in Maniatis et al., supra, page 109-112. Other isotopes which can be used in the nick translation labeling of DNA or in synthesis of labeled RNA are $^{35}$S, $^3$H and [125]I. Several non-radiometric labeling techniques are also applicable, including biotinylation of DNA as described by Chan et al., supra, and covalent attachment of reporter enzymes as described by Renz et al., supra.

Prior to labeling a DNA probe the cloned probe insert is excised from the vector by endonuclease digestion, then the probe DNA is ordinarily separated from the vector DNA by gel electrophoresis and eluted from the gel, as described in Vogelstein et al., Proc. Nat'l Acid. Sci, USA, 76:615-619 (1979) and Yang et al., Methods in Eznymology, 68:176-182 (1979). It is presently preferred to carry out at least one such gel electrophoresis prior to affinity removal of vector sequences, according to this invention, although it is contemplated that the method of the invention may in some cases permit elimination of the electrophoresis step.

After labeling dsDNA and contaminating vector by nick translation, the DNA is denatured to produce ssDNA by conventional means, e.g., by heating at a temperature of 90-100°C for several minutes or by mixing with dilute sodium hydroxide, about pH10 or a higher. Bloomfield et al., Physical Chemistry of Nucleic Acids, pages 332-339. The ssDNA is then ready for affinity purification in accordance with this invention.

If the probe DNA is labeled by means other than nick translation, e.g., by the method of Renz et al., supra, the labeling step can be performed after the denaturation step, and can even be performed after the affinity purification steps of this invention.

After synthesizing labeled RNA and separating the RNA from the DNA template and unincorporated rNTP's, by the methods described in Melton et al., supra, the RNA is ready for removal of contaminating vector sequences by the affinity purification method of this invention.

Affinity Purification

The removal of vector related or other undesired NA sequences is accomplished by the addition of capture NA (homologous to the vector sequences) to the NA probe where the capture NA is either attached to a bead support or attached to one member of a specific binding pair. Both the capture NA and probe NA must be in a single stranded form to allow efficient hybridization between the capture NA and the undesired probe NA. Hybridization conditions are selected which allow the undesired NA sequences to hybidize to the capture DNA. Preferred hybridization conditions include aqueous medium, a high salt buffer temperature of 65°C and reaction time of 1 hour or longer as illustrated below. Other hybridization conditions can be used, e.g., conditions disclosed in U.S. Patent 4,358,535, col. 5 and in Maniatis, Molecular Cloning, a Laboratory Manual, Cold Spring Harbor Laboratory, 1982, pages 208-209, 324-333 and 387-389. Generally, the hybridization will be carried out in aqueous buffered medium containing 0-60 volume percent of polar organic solvent such as formamide, at a temperature in the range of about 30 to 80°C, usually 40-70°C, with a reaction time of about 15 minutes to 5 hours, preferably 1-2 hours. Preferably, the hybridization is carried out in a microcentrifuge tube such as an Eppendorf tube, but it can be carried out in a plastic bag or other suitable container. Removal of the undesired NA sequences from the solution is achieved by removing the capture NA with the undesired NA sequences hybridized to it by the appropriate method. In the case of the capture NA attached directly to a head support the support can be pelleted by centrifugation or removed by the presence of a magnetic field when the beads are magnetic.

In the case of the specific binding pair technique the removal of the capture NA is achieved by the addition of the other member of the specific binding pair which is attached to a support, separation of the undesired NA hybridized to the capture NA is accomplished by the association of the binding pair followed by the removal of the capture support.

In the case where capture ssDNA is covalently attached directly to beads, the capture beads can be denatured to remove the hybridized vector ssNA and recycled. The process must be rigorous enough to remove the vector NA without destroying the beads or the crosslinking of the DNA to the beads. Treatment of the beads with heat, dilute alkali or organic solvents can accomplish this recycling.

Kits

This invention includes kits containing reagents and instructions for carrying out the method of the invention.

One type of kit will contain solid, water-soluble beads to which is covalently attached sequences of a DNA cloning vector, hybridization buffer and instructions. A second type of kit will contain: solid, water insoluble beads to which is covalently attached one member of a specific binding pair; capture ssNA to which is covalently attached the other member of the specific binding pair; hybridization buffer; and instructions. A preferred kit of the first type includes: a vial containing about 20 mg of aminosilane coated $CrO_2$ beads to which is covalently attached about 30 $\mu$g of ssDNA sequences of pBR322 and about 1 ml of a storage buffer (1.0 M Tris ● HCl, pH 7.5, 0.5% SDS); a second vial containing an additional 50 mL of storage/hybridization buffer; a third vial containing about 50 mL of recycling buffer (99% formamide 1XTE), and the following instructions:

VECTOR ADSORPTION USING CAPTURE BEADS

I. Reagents and Equipment

A) Storage/Hybridization Buffer - 1.0 m Tris ● HCl pH 7.5 0.5% SDS

B) Recycling Buffer - 99% Formamide IX TE

C) Shaking Water Bath (Labquake in Oven at 65° C)

II. Adsorption of Trace Vector Contamination from Nick Translated Probe

A) Spin beads in a microfuge for 2 min. and carefully remove storage/hybridization buffer so that beads are not disturbed -50 to 100 $\mu$l on top of the beads may be left. Discard buffer.

B) Add 1 ml of storage/hybridization buffer briefly agitate, spin beads for ~2 min. in a microfuge and carefully remove supernatant. Discard buffer.

C) Add 400 $\mu$l of storage/hybridization buffer to beads.

D) Boil probe for 5-10 min. Up to 1 $\mu$g of probe can be adsorbed at one time. Probe volume should be ≦100 $\mu$l. Add 20% more CPMs than you need (loss due to adsorption).

E) Immediately add the denatured probe to the beads and start agitation. Incubate at 65° C for at least 1 hr. Longer incubations are not detrimental.

F) After adsorbing the probe, spin for ~2 min. in a microfuge and carefully remove supernatant. Be careful not to remove beads at this point. If beads are disturbed, they can easily be recentrifuged.

G) Add 500 $\mu$l of storage/hybridization buffer to beads briefly agitate, spin beads out, carefully remove supernatant being careful not to pick up beads and add to previous aliquots.

H) Adsorbed probe can be boiled for 10 min. at this point. However, it has been shown to result in minimal improvement in sensitivity if at all.

I) Add preadsorbed probe to hybridization bag. Either premix with hybridization buffer or add directly to bag. You may want to check CPMs to determine recovery.

III. Recycling Beads

A) Add 1 ml of recycling buffer and incubate at 70° C for 5 min. Spin beads out and remove supernatant.

B) Repeat A.

To Reuse Immediately:

A) Add 1 ml of storage/hybridization buffer, spin beads out. Remove supernatant and discard.

B) Add 400 $\mu$l of storage/hybridization buffer to beads. Beads are now ready for probe addition.

To Store:

A) Wash IX with 1 ml of storage/hybridization buffer. Spin beads and discard buffer.

B) Add 1 ml of storage/hybridization buffer. Put beads in 4° C refrigerator until needed.

In the above Tris ● HCl means tris-(hydroxymethyl)aminomethane hydrochloride. SDS stands for sodium dodecyl sulfate, and 1X TE stands for 10 mM Tris and 1 mM EDTA, pH 7.5.

All patents, patent applications, journal articles and books cited in this application are incorporated herein in their entirety.

The invention will be further illustrated by the following examples.

## Example 1

### Bead Synthesis

150 mg of P-100 aminoethyl polyacrylamide beads were weighed out and transferred to a 15 ml corning polypropylene centrifuge tube. 8 mls of 0.2 morpholinoethanesulfonic acid (MES) pH 5.8 was added to the P-100 beads and allowed to hydrate for two hours at room temperature.

1.2 mgs of MSP I digest pBR322 and 200000 cpms of $^{32}$P 5' end labeled MSP I digested pBR322 were combined and ethanol precipitated. The pellet was rinsed once with 100% ethanol and dried. The DNA pellet was dissolved in 1 ml of 0.2 M MES pH 5.8. The DNA solution was then transferred into the bead suspension and the total volume was adjusted to 10 mls.

The coupling agent 1-ethyl-3,3-dimethylamino propylcarbodimide (EDAC) was weighted out. 200 mgs of EDAC was added to the bead, DNA mixture. The coupling reaction was allowed to run overnight at room temperature with agitation.

After the coupling reaction the uncoupled DNA was removed and the DNA attached to the support was denatured by first removing the reaction buffer and then washing the beads four times with 0.1N NaOH 0.1% SDS, 5 mls each wash. The washes and reaction buffer were saved for quantitation of the coupling efficiency. 84% of the pBR322 was coupled to the beads.

5 mls of 0.3M NaOAc pH 5.0 was added to the beads to neutralize the wash buffer. The pBR beads were then suspended in 0.1 M NaPhosphate pH 7.5 0.1% SDS for storage.

### Vector Adsorption

To demonstrate the effectiveness of pBR beads in removing pBR vector from gel isolated DNA probes, a comparison of adsorption treated probe and untreated probe was performed. The system used was HIND III T fragment of human CMV (Fleckenstein, B., I. Muller & J. Collins 1982 Cloning of the Complete Human Cytomegalouirus Genome in Cosmids. Gene 18: 39-46) as $^{32}$P labeled nick translated DNA probe hybridized to a STD curve (various concentrations) of CMV DNA and pBR322 DNA immobilized on a membrane. The STD curve of CMV DNA was used to show the hybridizability of the HIND III T DNA probe to its target and the pBR322 DNA STD curve was used to demonstrate the reduction in crossreactivity of the HIND III T probe to pBR322-like sequences.

CMV DNA was immobilized on nitrocellulose membranes in a concentration range $10^8$, $10^7$, $10^6$, $10^5$ copies (molecules)/well. The pBR322 was immobilized in the same manner in a concentration range from $10^9$, $10^8$, $10^7$, $10^6$ copies/well.

The HIND III T CMV DNA probe was produced from HIND III T CMV fragment cloned into pBR322, transformed and grown in an E. coli cell line. The HIND III T plasmid was isolated from the growth, digested with HIND III restriction enzyme and isolated on an agarose gel. The gel isolated HIND III T DNA fragment was $^{32}$P nick translated to a specific activity of $3.1 \times 10^8$ cpms/μg using modified technique of Rigby, et al. (1977) J. Mol. Biol. 113, 237-251.

Bead adsorption of vector sequences was performed on a portion of the nick translated HIND III T. This was carried out by adding $5 \times 10^7$ cpms of probe to approximately 15 mgs of vector adsorption beads in 0.5 mls of 1M NaPHOS pH 7.5 1% SDS. The reaction was then incubated at 65°C for one hour with agitation.

The probe was removed from the beads by spinning down the adsorption beads and removing the supernatant containing the probe. 0.5 mls of 1.0 M NaPHOS pH 7.5 1% was added to the beads. The beads were resuspended and again centrifuged out and the supernatant was added to the first aliquots of probe removed.

Two membrane sets, each set containing a CMV and pBR322 STD curve, were prehybridized for two hours at 65°C in 5X SSPE, 0.5% SDS, 5X Denhardt's, 100 mg/ml salmon sperm DNA. The nonadsorbed and adsorbed Hind III T probes were boiled for five minutes to denature the probe. The probes were added to the separate hybridization pouches at a concentration of $1 \times 10^7$ cpms/ml and incubated overnight at 65°C.

After hybridization, the membrane were washed to remove unhybridized probe. This was carried out by: (1) washing 10 minutes at room temperature in 0.1X SSPE, 0.1% SDS, (2) washing 30 minutes at 60°C in 0.1X SSPE, 0.1% SDS, (3) washing 10 minutes at room temperature in 0.1% SSPE, 0.1% SDS. The membranes were dried and autoradiographed at -70°C.

The comparison shows a 50 to 100 fold reduction in the pBR322 signal with adsorption of vector and no reduction in the sensitivity of the HIND III T probe.

## EXAMPLE 2

pBR322 vector contamination can also be removed from recombinant NA by using solution hybridization of the NA sample with complementary pBR322 DNA containing one member of a specific binding pair (e.g. biotin) followed by the selective removal of the hybridized pBR322 sequences with the second member of the specific binding pair (e.g. streptavidin) which has been covalently attached to a solid support.

Plasmid pBR322 DNA was biotinylated by nick translation with Bio-11-dUTP, purified by phenol/chloroform extraction and ethanol precipitated. Streptavidin was covalently attached to aminosilane coated $CrO_2$ magnetic particles by standard crosslinking with glutaraldehyde. The HIND III L CMV DNA probe was produced from the HIND III L fragment of CMV DNA cloned into pBR322, transformed and grown in an E. coli cell line. The HIND III L plasmid was isolated from the growth, digested with HIND III restriction enzyme and isolated on an agarose gel. The gel isolated HIND III L fragment was $^{32}P$ nick translated to a specific activity of $3 \times 10^8$ cpm/Hg using a modified technique of Rigby, et al. (1977) J. Mol. Biol. 113, 237-251.

The effectiveness of the specific binding pair interaction to remove pBR322 contamination was evaluated by comparing treated and untreated CMV-L probe solution after hybridization with identical nylon membranes containing immobilized CMV-L DNA ($10^8$, $10^7$, $10^6$, $10^5$ copies/well) and pBR322 DNA ($1 \times 10^9$, $2 \times 10^8$, $1 \times 10^8$, $5 \times 10^7$, $2 \times 10^7$ copies/well).

CMV-L probe ($1.2 \times 10^7$ cpm in 24 μL of 20% ethanol) was added to biotinylated pBR322 DNA (2μg) in 400 μL of 1.0 M $Na_2HPO_4$, pH = 7.5, 0.5% SDS, the entire solution was denatured in a boiling water bath for 7 minutes, chilled quickly on ice and brought up to 65° C in a water bath for 1 hour. To the hybridization solution was added a suspension of streptavidin-$CrO_2$ particles (125 μg in 150 μL) and the resulting suspension was incubated at room temperature for 30 minutes followed by 30 minutes at 37° C. The streptavidin-$CrO_2$ particles bearing biotinylated hybrids were removed by centrifugation and the treated probe supernatant was diluted to 1 mL with 1.0 M $Na_2HPO_4$, pH = 7.5, 0.5% SDS and added to a nylon membrane with immobilized target DNA that had been prehybridized in 9 mL of 10X Denhardt's, 1 M NaCl, 1% SDS, 0.05 M TRIS.HCl pH = 7.5, 0.1% sodium pyrophosphate, 10% dextran sulfate, and 100 μg/mL of denatured salmon sperm DNA.

Untreated CMV-L DNA probe ($1.2 \times 10^7$ cpm in 24 μL of 20% ethanol) was diluted to 1 mL with 1.0 M $Na_2HPO_4$, pH = 7.5, 0.5% SDS, denatured by heating in a boiling water bath for 7 minutes, chilled on ice and added to a nylon membrane with immobilized target DNA that had been prehybridized in 9 mL of 10X Denhardt's, 1 M NaCl, 1% SDS, 0.05 M TRIS.HCl pH = 7.5, 0.1% sodium pyrophosphate, 10% dextran sulfate, and 100 μg/mL of denatured salmon sperm DNA.

A suitable control would be to treat the contaminated CMV-L DNA probe with 2 μg of pBR322 (non-biotinylated) in the same manner as described for the biotinylated pBr322 (above) for hybridization with target DNA on a nylon membrane.

After hybridization at 68° C for 16 hours, the nylon membranes were washed twice at room temperature for 10 minutes with 2X SSC, twice at 68° C for 30 minutes with 2X SSC, 1% SDS, and twice at room temperature for 10 minutes with 0.2X SSC. The washed membranes were dried and autoradiographed at -70° C for 16 hours and comparison of the treated and untreated probe showed a 5 fold reduction in the pBR322 crossreactivity for the treated probe and equal sensitivity for detection of the CMV-L target DNA.

## Claims

1. A method of removing undesired ssNA from a sample containing desired and undesired ssNA in an aqueous medium which comprises (a) contacting the sample under hybridization conditions with capture ssNA which is complementary to the undesired ssNA and which is covalently bound to solid, water insoluble beads or to one member of a specific binding pair, (b) in the case where the capture ssNA is covalently bound to one member of a specific binding pair, contacting the sample with solid, water insoluble beads to which is covalently bound the other member of the specific binding pair, and (c) separating the beads carrying hybridized undesired NA from the liquid medium.

2. Method of claim 1 wherein the undesired ssNA comprises labeled ssDNA or ssRNA contaminating sequences derived from a DNA cloning vector, the desired ssNA comprises a labeled ssDNA or ssRNA probe derived from a DNA insert which has been cloned in the vector; and the capture ssNA comprises ssDNA sequences of the cloning vector.

3. Method of claim 2 wherein the vector is of bacterial or viral origin.

4. Method of claim 3 wherein the DNA insert is derived from an infectious agent.

5. Method of any of claims 1-4 wherein the capture ssNA is covalently bonded to the solid, water insoluble beads.

6. Method of any of claims 1-4 wherein the capture ssNA is covalently bonded to one member of a specific binding pair.

7. Method of claim 6 wherein the capture ssNA is covalently bound to biotin and the solid, water insoluble beads are covalently bound to avidin or streptavidin.

8. In a method of making a labeled ssDNA probe which comprises

(a) cloning recombinant DNA comprising probe DNA insert in a DNA cloning vector,

(b) excising the probe DNA from the cloning vector by treatment with endonuclease,

(c) labeling the probe DNA and

(d) denaturing the probe and vector DNA,

the improvement which comprises (e) contacting the denatured probe and vector DNA in aqueous medium, under hybridization conditions, with capture ssDNA sequences of the cloning vector which are covalently bound to solid, water insoluble beads or to one member of a specific binding pair, (f) in the case where the capture ssDNA is covalently bound to one member of a specific binding pair, contacting the latter with solid, water insoluble beads to which is covalently bound the other member of the specific binding pair, and (g) separating the beads carrying hybridized vector DNA from the aqueous medium.

9. Method of claim 8 wherein prior to labeling and denaturing steps (c) and (d) the probe and vector DNA are subjected to gel electrophoresis to remove the bulk of the vector DNA from the probe DNA.

10. Method of claim 9 wherein labeling step (c) is performed before denaturing step (d) and wherein the probe and vector DNA are labeled by nick translation using radiolabeled or biotin-labeled dNTP's.

11. Method of claim 10 wherein the cloning vector is a bacterial origin vector and the probe comprises ssDNA sequences of an infectious agent.

12. In a method of making a labeled ssRNA probe which comprises:

(a) cloning recombinant DNA comprising a DNA insert, corresponding to the desired ssRNA probe, in a DNA cloning vector having a transcription promoter upstream of the insert,

(b) cutting the cloned DNA with endonuclease to produce linear cloned DNA

(c) contacting the linear cloned DNA with labeled rNTP's in presence of RNA polymerase to synthesize the labeled ssRNA probe and

(d) separating the DNA and unincorporated rNTP's from the RNA,

the improvement which comprises (e) contacting the RNA in aqueous medium, under hybridization conditions, with capture ssDNA sequences of the cloning vector which are covalently bound to solid, water insoluble beads or to one member of a specific binding pair, (f) in the case where the capture ssDNA is covalently bound to one member of a specific binding pair, contacting the latter with solid, water insoluble beads to which is covalently attached the other member of the specific binding pair, and (g) separating from the aqueous medium the beads carrying hybridized vector-derived RNA.

13. Method of claim 12 wherein the cloning vector is a bacterial origin vector and the probe comprises ssRNA sequences transcribed from DNA of an infectious agent.

14. Method of claim 11 or 13 wherein the capture ssDNA sequences are covalently bound to the beads.

15. Method of claim 14 wherein the beads are magnetic.

16. Method of claim 15 wherein the beads are silane-coated $CrO_2$ beads.

17. Method of claim 11 or 13 wherein the capture ssDNA sequences are bonded to one member of a specific binding pair.

18. Method of claim 17 wherein the capture ssDNA sequences are bonded to biotin and the solid, water insoluble beads are bonded to avidin or streptavidin.

19. Solid, water-insoluble beads to which is covalently attached sequences of a DNA cloning vector.

20. Beads of claim 19 wherein the sequences are ssDNA sequences complementary to both strands of a DNA cloning vector.

21. Beads of claim 20 wherein the sequences are sequences of a bacterial-origin vector.

22. Beads of claim 21 wherein the NA sequences are sequences of PBR322.

23. Beads of claims 19, 20, 21 or 22 composed of ferromagnetic material.

24. Beads of Claim 23 which are composed of silane-coated $CrO_2$.

25. A kit comprising beads of claim 19, buffers, and printed instructions for using the contents to remove contaminating vector sequences from a labeled, recombinant cloned ssNA probe.

26. Compounds consisting of sequences of a DNA cloning vector covalently attached to one member of a specific binding pair.

27. Compounds of claim 25 wherein the DNA sequences are ssDNA sequences.

28. Compounds of claim 26 wherein the ssDNA sequences are covalently attached to biotin.

29. Compounds of claim 27 wherein the ssDNA sequences are sequences of a bacterial origin vector.

30. Compounds of claim 28 wherein the ssDNA sequences are sequences of pBR322.

31. A kit comprising compounds of claim 26, solid water insoluble beads to which is covalently attached the other member of the specific binding pair, buffers, and printed instructions for using the contents to remove contaminating vector sequences from a labeled recombinant NA probe.

32. A kit comprising compounds of claim 29, solid water insoluble beads to which is covalently attached avidin or streptavidin, buffers, and printed instructions for using the contents to remove contaminating vector sequences from a radiolabeled recombinant cloned ssNA probe.